(19) Europäisches Patentamt
European Patent Office
Office européen des brevets

(11) Veröffentlichungsnummer: **0 134 464 B1**

# (12) EUROPÄISCHE PATENTSCHRIFT

(45) Veröffentlichungstag der Patentschrift: **20.04.88**

(51) Int. Cl.⁴: **C 07 C 11/02**, C 07 C 11/12, C 07 C 43/04, C 07 C 43/11

(21) Anmeldenummer: **84107641.7**

(22) Anmeldetag: **02.07.84**

(54) **Verfahren zur Herstellung von 3,3- bzw. 2,3-Dimethylbuten und 2,3-Dimethylbutadien bzw. zur gleichzeitigen Herstellung dieser Olefine und Glykol- bzw. Polyglykol-n-alkyl-3,3- bzw. -2,3-dimethylbutylether aus Mono- und Dichlor-dimethylbutan.**

(30) Priorität: **25.08.83 DE 3330599**
**02.03.84 DE 3407756**

(43) Veröffentlichungstag der Anmeldung:
**20.03.85 Patentblatt 85/12**

(45) Bekanntmachung des Hinweises auf die Patenterteilung:
**20.04.88 Patentblatt 88/16**

(84) Benannte Vertragsstaaten:
**AT BE CH DE FR GB IT LI NL**

(56) Entgegenhaltungen:
**DE - B - 1 253 700**
**FR - A - 1 563 517**
**US - A - 3 607 965**

**ACTA CHEMICA SCANDINAVICA, Band 13, Teil I, 1959, Seiten 611-613, Kopenhagen, DK; A. BRANDSTRÖM : "A method for the preparation of trialkylacetaldehydes"**

(73) Patentinhaber: **HÜLS AKTIENGESELLSCHAFT, - RSP Patente / PB 15 - Postfach 13 20, D-4370 Marl 1 (DE)**

(72) Erfinder: **Kaufhold, Manfred, Dr., Jasminweg 20, D-4370 Marl (DE)**
Erfinder: **Smolka, Werner, Brassertstrasse 35 b, D-4370 Marl (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Die Erfindung betrifft ein Verfahren zur Herstellung von 3,3-Dimethylbuten-1 ( = Neohexen), 2,3-Dimethylbuten-1 und 2,3-Dimethylbuten-2 oder von 2,3-Dimethylbutadien bzw. zur gleichzeitigen Herstellung dieser Olefine und Glykol- bzw. Polyglykol-n-alkyl-3,3- bzw. -2,3-dimethylbutylether der Formel 1 ($R_2$ = 3,3- bzw. 2,3-Dimethylbutyl) durch Dehydrochlorierung von 1-Chlor-3,3-dimethylbutan bzw. 1-Chlor-2,3-dimethylbutan allein oder im Gemisch mit 2-Chlor-2,3-dimethylbutan bzw. von Dichlor-2,3-dimethylbutan mit Alkalihydroxiden in Gegenwart von Glykol- bzw. Polyglykolethern der allgemeinen Formel 1

$$R_1(OCH_2CH_2)_nOR_2 \qquad (1)$$

$R_1$ = n-Butyl
n-Hexyl
n-Octyl
2-Ethylhexyl
n = 1, 2, 3
$R_2$ = t-Butyl
= 3,3-Dimethylbutyl bzw.
= 2,3-Dimethylbutyl

Synthesen von 3,3-Dimethylbuten-1 (Neohexen) aus 1-Chlor-3,3-dimethylbutan (Neohexylchlorid) sind in der Literatur beschrieben, wie z.B. in der DE-PS 1 253 700, die eine thermische Dehydrochlorierung bei 350 bis 800 (500 bis 700) °C und kurzen Verweilzeiten in Abwesenheit von Katalysatoren vorschlägt. Dieses Verfahren ist wegen der hohen Temperaturen und des entstehenden korrosiven Chlorwasserstoffs nur mit grossem technischem Aufwand realisierbar. Ausserdem muss die Verweilzeit sehr genau geregelt werden, damit nicht durch Umlagerung des 3,3-Dimethylbuten-1 (Neohexen) 2,3-Dimethylbuten-1 und 2,3-Dimethylbuten-2 in grösseren Mengen entstehen.

Sodatova et al. (s. Chemical Abstr. Vol. 79 (1973), S. 41796) schlagen vor, 1-Chlor-3,3-dimethylbutan (Neohexylchlorid) mit Kaliumacetat in Gegenwart von Essigsäure bei 180 bis 200°C und 22 Atmosphären zum entsprechenden Essigsäureester umzusetzen und diesen thermisch bei 500°C zum 3,3-Dimethylbuten-1 (Neohexen) und Essigsäure zu spalten. Auch dieser zweistufige Prozess erfordert hohe Temperaturen und ist nur in einer Druckapparatur durchführbar.

Dagegen wird von A. Brändström (s. Acta Chem. Scand. 13 611 bis 12 (1959) eine Labormethode zur Dehydrochlorierung von Neohexylchlorid vorgestellt, die einstufig bei relativ niedrigen Temperaturen, 120 bis 190°C, mit Kaliumhydroxid als Base und Polyethylenglykol als Lösemittel arbeitet. Mit Hilfe des eingesetzten Polyethylenglykols erzielt man bei diesem Verfahren eine homogene Mischung. Nachteilig ist aber hierbei, dass das entstehende Kaliumchlorid nicht auf technisch einfache Weise wie z.B. durch Wasserwäsche ohne Abwasserprobleme entfernt werden kann. Ausserdem ist die Ausbeute gering.

In der FR-A 1 563 517 werden Polyglykolether und Monoalkylether von Polyglykolen eingesetzt d.h. Verbindungen der allgemeinen Formel

$$R-O-(CH_2CH_2O)_nCH_2CH_2OH$$

Diese Verbindungen sind völlig oder zum grössten Teil wasserlöslich. Deshalb ist ihre Abtrennung von der entstehenden wässrigen Alkalichloridlösung sehr aufwendig, wenn man reines Abwasser erhalten will und diese teuren Verbindungen zurückgewinnen muss.

Auch Synthesen von 2,3-Dimethylbuten-1 und 2,3-Dimethylbuten-2 sind in der Literatur beschrieben, wie z.B. in der DE-AS 2 917 779, die eine dreistufige Synthese, ausgehend vom Isovaleraldehyd, vorschlägt. Bei diesem Verfahren wird Isovaleraldehyd mit Formaldehyd zum α-Isopropylacrolein umgesetzt, dieser ungesättigte Aldehyd wird anschliessend zum 2,3-Dimethylbutanol hydriert, und die darauf folgende Dehydratisierung liefert ein Gemisch der isomeren Olefine 2,3-Dimethylbuten-1 und 2,3-Dimethylbuten-2. Dieses Verfahren benötigt relativ kostspielige Chemikalien und ist wegen der vielen Stufen technisch aufwendig.

Trotz dieser Nachteile ist dieses Verfahren, wie in der Beschreibung der DE-AS 2 917 779 ausführlich dargelegt wird, besser als die in der Literatur zuvor bekannten Verfahren zur Dimerisation von Propen, bei denen die schwer abtrennbaren Methylpentene stets als Nebenprodukt anfallen.

Neben diesen Verfahren wurde auch die selektive Hydrierung des 2,3-Dimethylbutadiens zur Herstellung von 2,3-Dimethylbuten-2 vorgeschlagen. Dieser Ausgangsstoff ist aber schwer zugänglich, und deshalb ist dieses Verfahren für eine technische Realisierung von untergeordnetem Interesse.

Interessanter sind dagegen Verfahren, die vom leicht zugänglichen 2,3-Dimethylbutan ausgehen. Dieser Kohlenwasserstoff fällt bei der Produktion des Moschusriechstoffs, 7-Acetyl-1,1,3,4,4,6-hexamethyl-1,2,3,4-tetrahydronaphthalin (sog. Acetyl HMT, DE-PS 2 457 550), als Nebenprodukt an, und zwar bei einer bestimmten Stufe dieser Synthese, bei der Umsetzung von p-Cymol mit 3,3-Dimethylbuten-1 (Neohexen) oder mit 2,3-Dimethylbuten-1 in äquimolaren Mengen (DE-AS 1 035 826). Der Mechanismus dieser interessanten Reaktion wurde von T.F. Wood und J. Angioloni in Tetrahedron Letters No. 1, Seiten 1 bis 8 (1963) ausführlich beschrieben.

Die Umwandlung des 2,3-Dimethylbutans in das Olefingemisch ist beispielsweise durch Dehydrierung oder durch Chlorierung und anschliessende Dehydrochlorierung möglich. Die Dehydrierung, die von L.B. Fisher, M.P. Terpugova und I.L. Kotlyarevskii (Izvest. Vostoch Filial, Akad. Nauk, S.S.S.R. 1957, No. 9.53-6 oder Chem. Abstr. (1958) 12746) untersucht wurde, hat den

Nachteil, dass hohe Temperaturen von ca. 550°C erforderlich sind und dass 2,3-Dimethylbutadien als Nebenprodukt entsteht. Die Abtrennung des Diens und des nicht umgesetzten 2,3-Dimethylbutans von dem Monoolefingemisch ist wegen der sehr nahe nebeneinander liegenden Siedepunkte technisch sehr aufwendig.

Dieser Nachteil entfällt, wenn man ein Verfahren wählt, bei dem 1-Chlor-2,3-dimethylbutan und/oder 2-Chlor-2,3-dimethylbutan als Zwischenprodukte auftreten, da diese erheblich höher sieden als das 2,3-Dimethylbutan.

Die Herstellung eines Gemisches dieser Chlorverbindung ist durch Chlorierung des 2,3-Dimethylbutans technisch einfach zu realisieren. Allerdings entstehen hierbei in geringer Menge auch höher chlorierte Produkte. Das eigentliche Problem ist aber die anschliessende Dehydrochlorierung, da die beiden isomeren Monochlorverbindungen eine sehr unterschiedliche Stabilität zeigen. Nach Vives, V.C.; Kruse, C.W. und Kleinschmidt, R.F. (Ind. Engng. Chem., Product Res. Development 8 (1969) 4, 432 bis 435) wird 2-Chlor-2,3-dimethylbutan als tertiäre Chlorverbindung bereits bei ihrem Siedepunkt (Kp = 112°C) mit einer ganzen Reihe von Katalysatoren dehydrochloriert, während die primäre Chlorverbindung, das 1-Chlor-2,3-dimethylbutan Temperaturen um 300°C zur Dehydrochlorierung benötigt. Deshalb schlagen diese Autoren vor, als Chlorierungsagenz tert.-Butylchlorid einzusetzen, weil hierbei nur die tert.-Chlorverbindung entsteht. Aus dem tert.-Butylchlorid entsteht Isobutan, und zwar in äquimolaren Mengen als Abfallprodukt.

Das Verfahren ist also kostspielig wegen seines hohen Chemikalienverbrauchs. Im anfallenden Olefingemisch liegt das Isomerenverhältnis α-Olefin: β-Olefin bei 1:3.

Um eine weitgehende Dehydrochlorierung des Gemisches der primären und tertiären Chlorverbindung zu erreichen, wird in der US-PS 2 613 233 vorgeschlagen, eine zweistufige Spaltung durchzuführen, wobei in der ersten Stufe Temperaturen von 120 bis 130°C und in der zweiten Stufe Temperaturen von 500 bis 600°C eingestellt werden. Auch dieses Verfahren ist also relativ aufwendig und benötigt wegen der hohen Temperaturen teure Reaktoren aus Spezialstählen.

Alle bekannten Verfahren benötigen somit hohe Temperaturen, aufwendige technische Apparaturen oder kostspielige Chemikalien, sind nur in mehreren Stufen durchführbar oder bringen Probleme der Abfallbeseitigung.

Wünschenswert ist ein Verfahren, bei dem das technisch durch Umsetzung von tert.-Butylchlorid mit Ethylen leicht zugängliche 1-Chlor-3,3-dimethylbutan (Neohexylchlorid) bzw. das durch Chlorierung von 2,3-Dimethylbutan leicht zugängliche Gemisch der Monochloride als auch die als Nebenprodukt anfallenden dichlorierten Produkte in einfacher Weise dehydrochloriert werden, ohne kostspielige Chemikalien zu benötigen.

Es besteht daher ein grosses Interesse an einem Verfahren, nach dem man bei geringem technischen Aufwand aus dem kostengünstigen 1-Chlor-3,3-dimethylbutan (Neohexylchlorid) und den kostengünstigen Monochlorierungsprodukten des 2,3-Dimethylbutans, aus dem 2-Chlor-2,3-dimethylbutan und dem 1-Chlor-2,3-dimethylbutan, 3,3-Dimethylbuten-1 bzw. ein Gemisch aus 2,3-Dimethylbuten-1 und 2,3-Dimethylbuten-2 herstellen kann und mit dem man die höher chlorierten Produkte des 2,3-Dimethylbutans, das sind vorwiegend die isomeren Dichlor-2,3-dimethylbutane, ebenfalls dehydrochlorieren und die daraus resultierenden Reaktionsprodukte einer Verwertung zuführen kann.

Die sich hieraus ergebende Aufgabe wird erfindungsgemäss entsprechend den Angaben der Patentansprüche gelöst.

Überraschenderweise erhält man 3,3-Dimethylbuten-1 (Neohexen) in hoher Reinheit (über 98%) bzw. ein Gemisch an 2,3-Dimethylbuten-1 und 2,3-Dimethylbuten-2 mit einer Reinheit von über 98% (z.B. gaschromatographisch ermittelt), in einem Isomerenverhältnis von α-: β-Olefin wie ca. 14:3 und in guten Ausbeuten, indem man festes Alkalihydroxid und einen Glykol- bzw. Polyglykolether vorlegt, auf 100 bis 250°C, vorzugsweise 120 bis 200°C, insbesondere 150 bis 180°C, erwärmt, die Monochloride des 3,3- und des 2,3-Dimethylbutan-1 bzw. das Gemisch der Monochloride des 2,3-Dimethylbutans (im folgenden bezeichnet als Monochloridgemisch) zugibt und die anfallenden Leichtsieder und Wasser und nichtumgesetzte Chlorverbindungen über eine Kolonne abdestilliert. Die Umsetzung erfolgt im allgemeinen in einer Zeit von 2 bis 10 Stunden.

Als Alkalihydroxide sind vorzugsweise Natrium- oder Kaliumhydroxid sowie deren Mischungen geeignet. Das Molverhältnis Alkali : Chlorverbindung beträgt im allgemeinen 1:1 bis 1:5, bevorzugt 1:1 bis 1:2. Geeignete Glykol- bzw. Polyglykolether sind Glykol- bzw. Polyglykol-di-alkylether der Formel 1, beispielsweise Glykol-n-butyl-3,3- bzw. -2,3-dimethylbutylether, Diglykol-n-butyl-3,3- bzw. -2,3-dimethylbutylether, Diglykol-n-butyl-tert.-butylether, Triglykol-n-butyl-3,3-bzw. -2,3-dimethylbutylether.

Die Glykol- bzw. Polyglykolether setzt man im allgemeinen in Mengen von 0,1 bis 1 Mol/Mol der Chlorverbindung ein, vorzugsweise von 0,25 bis 0,5 Mol/Mol.

Beim Einsatz der Glykol- bzw. Polyglykol-n-butyl-3,3- bzw. -2,3-dimethylbutylether erfolgt die Umsetzung vorzugsweise bei Temperaturen von 150 bis 250°C, insbesondere von 200 bis 220°C.

Als Glykol- bzw. Polyglykolether sind insbesondere solche geeignet, die nicht gut in Wasser bzw. wässriger Alkalichloridlösung löslich sind, so dass die als Nebenprodukt entstehenden Alkalichloride durch Wasserwäsche entfernt werden können und das Abwasser einen geringen Kohlenstoffgehalt hat. Es hat sich überraschenderweise gezeigt, dass diese relativ unpolaren Lösemittel die Reaktion zwischen Alkalihydroxid und dem Monochloridgemisch ermöglichen.

Ein für dieses Verfahren besonders geeigneter

Polyglykoldialkylether ist z.B. der Diglykol-n-butyl-tert.-butylether, der technisch durch Umsetzung von n-Butyl-diglykol mit Isobutylen zugänglich ist.

Überraschenderweise hat sich gezeigt, dass ein für das erfindungsgemässe Verfahren geeignetes Lösungsmittel bei dem Verfahren selbst erzeugt wird, wenn man zunächst einen Glykol- bzw. Polyglykolmonoalkylether der Formel 2 als Lösemittel bzw. Suspensionsmittel einsetzt. Das Verfahren kann dann zweistufig durchgeführt werden und liefert in beiden Stufen das 3,3-Dimethylbuten-1 bzw. die 2,3-Dimethylbutene und in der ersten Stufe den für die zweite Stufe als Löse- bzw. Suspensionsmittel geeigneten Ether.

Zu dieser Herstellung der neuen Dialkylether sind als Glykol- bzw. Polyglykolmonoalkylether zwar viele verschiedene Verbindungen einsetzbar, beispielsweise die obengenannten Ethylenoxidaddukte, aus wirtschaftlichen Gründen ist den auf dem Markt leicht zugänglichen Umsetzungsprodukten des n-Butanols mit Ethylenoxid der Vorzug zu geben; das sind folgende Verbindungen:

n-Butylglykol
n-Butyldiglykol
n-Butyltriglykol

Der Einsatz dieser Glykolether bietet u.a. folgende Vorteile:

1. Trotz ihrer guten Löslichkeit in Wasser enthält das bei dem erfindungsgemässen Verfahren anfallende Abwasser relativ wenig Kohlenstoffverbindung.

2. Die in der ersten Stufe entstehenden Ether vom Typ der Formel 1 mit $R_2$ = 3,3- bzw. 2,3-Dimethylbutyl fallen bei einfacher destillativer Aufarbeitung in reiner Form an und sind für die 2. Stufe des Verfahrens geeignet.

3. Die unter Punkt 2 genannten Ether sind thermisch und gegen Säuren stabiler als die obengenannten entsprechenden tert.-Butylether, so dass bei dem Verfahren nur mechanische Verluste an Suspensionsmitteln auftreten.

Diese Ergebnisse sind in ihrem Ausmass überraschend und zeigen die einfache technische Durchführbarkeit des Verfahrens. Die Ether mit der Formel 1 mit $R_2$ = 3,3- bzw. 2,3-Dimethylbutyl sind neu.

Es handelt sich insbesondere um folgende neue Dialkylether: Glykol-n-butyl-3,3- bzw. -2,3-dimethylbutylether, Diglykol-n-butyl-3,3- bzw. -2,3-dimethylbutylether, Triglykol-n-butyl-3,3- bzw. -2,3-dimethylbutylether.

Aufgrund ihrer überraschend grossen Stabilität eignen sie sich auch für andere anwendungstechnische Zwecke, wie z.B. als Brems- und Hydraulikflüssigkeiten, Kühl- und Heizmedien, Schmiermittel, selektive Adsorptionsmittel für saure Gase usw.

Ein weiterer Vorteil dieses Verfahrens ist, dass man auch die höher chlorierten Produkte des 2,3-Dimethylbutans, das Dichlor-2,3-dimethylbutan, dehydrochlorieren kann, so dass man sie nicht auf kostspielige Weise, wie z.B. mittels Verbrennen vernichten muss. Als leichtsiedendes Spaltprodukt entsteht 2,3-Dimethylbutadien, das ein wertvolles Ausgangsprodukt für andere Synthesen darstellt.

Auch das oben erwähnte hohe Verhältnis der 2,3-Dimethylbutene ist vorteilhaft, weil auf diese Weise 2,3-Dimethylbuten-1 in grossen Mengen auf wirtschaftliche Weise zugänglich wird.

Dieses Olefin ist ein geeigneter Reaktionspartner des p-Cymols bei der Herstellung des oben erwähnten Moschusriechstoffs Acetyl-HMT (DE-AS 1 593 653), so dass über diesen Weg das als Nebenprodukt anfallende 2,3-Dimethylbutan in die Produktion des Acetyl-HMT zurückgeführt werden kann.

Das erfindungsgemässe Verfahren führt man beispielsweise folgendermassen durch:

Ausgangsprodukt des erfindungsgemässen Verfahrens sind 1-Chlor-3,3-dimethylbutan, das man im allgemeinen aus tert.-Butylchlorid und Ethylen mit Aluminiumchlorid als Katalysator bei tiefen Temperaturen, −15 bis −30°C herstellt (Schmerling, J. Amer. Chem. Soc., 67, 1152 (1945), bzw. chlorierte 2,3-Dimethylbutane, die in üblicher Weise mit oder ohne Katalysator bei etwas erhöhter Temperatur, z.B. bei 50°C durch Chlorieren von 2,3-Dimethylbutan erhalten werden, wobei ein Austausch von Wasserstoff gegen Chlor stattfindet (Houben Weyl, Methoden der organischen Chemie, Band V/3 Halogenverbindungen (1962) S. 571 ff, 4. Auflage, Georg Thieme Verlag, Stuttgart). Das Chlorierungsprodukt wird durch Destillation in zwei Fraktionen aufgeteilt. Die erste Fraktion ist nicht umgesetztes 2,3-Dimethylbutan, das in die Chlorierung zurückgeführt wird. Die zweite Fraktion ist das gewünschte Monochloridgemisch, 2-Chlor-2,3-dimethylbutan und 1-Chlor-2,3-dimethylbutan, die im Verhältnis von 35:65 bis ca. 40:60 anfallen. Der Destillationsrückstand enthält die höher chlorierten Produkte, wobei erwartungsgemäss die Dichloride überwiegen. Die Monochlorverbindung und die Dichlorverbindung werden getrennt in die Dihydrochlorierungsstufe eingesetzt, und zwar in folgender Weise:

In einer üblichen Rührapparatur, auf der sich eine Destillationskolonne befindet, wird Alkalihydroxid, z.B. festes Natriumhydroxid oder Kaliumhydroxid oder Gemische dieser Alkalien und ein n-Butylglykol- bzw. -polyglykoldi-ether vorgelegt, auf 100 bis 250°C, vorzugsweise 200 bis 220°C, unter Rühren erwärmt, wobei sich zwei flüssige Phasen bilden und das Monochloridgemisch oder das Dichloridgemisch langsam zugegeben wird. Höhere Temperaturen als 250°C sollten vermieden werden, weil dann die Gefahr einer Zersetzung des Butylglykolethers besteht. Bei Temperaturen unter 150°C verläuft die Reaktion langsam.

Wenn ein Teil der Chlorverbindung zugegeben ist, beobachtet man die Bildung von feinen Alkalichlorid-Kristallen, und am Kopf der Kolonne fällt bei ca. 40°C bis 85°C die entsprechende ungesättigte Verbindung an. Nach einiger Zeit steigt die

Kopftemperatur bis auf 80 bis 100°C, und jetzt fallen in der Destillationsvorlage grössere Mengen an Wasser an. Gegen Schluss steigt die Kopftemperatur je nach Molverhältnis Alkali zu eingesetzter Chlorverbindung bis auf die Siedetemperatur des Einsatzproduktes, z.B. 117°C beim 1-Chlor-3,3-dimethylbutan. Das Molverhältnis Alkali : Chlorverbindung wählt man zweckmässigerweise 1:1 bis 1:5, vorzugsweise 1:1 bis 1:2. Da das nicht umgesetzte Alkali nicht zurückgewonnen wird, ist es nicht zweckmässig, die Chlorverbindung im Unterschuss einzusetzen.

Wenn kein Destillat mehr anfällt, wird unter Rühren abgekühlt und dann mit Wasser das fein suspendierte Alkalichlorid herausgewaschen. Die Ölphase wird destilliert und hierbei die entsprechenden Glykol-n-butyl-bzw. Polyglykol-n-butyl-3,3-bzw. -2,3-dimethylbutyl-ether zurückgewonnen.

Von den zweiphasigen Destillaten wird das Wasser abgetrennt und verworfen. Die Redestillation der Ölphase liefert die Monoolefine in über 98%iger Reinheit und das 2,3-Dimethylbutadien in über 97%iger Reinheit.

Das erfindungsgemäss hergestellte 3,3-Dimethylbuten-1 und das 2,3-Dimethylbuten-1 eignen sich zur Herstellung des Acetyl-HMT (ein Moschusriechstoff, 7-Acetyl-1,1,3,4,4,6-hexamethyl-1,2,3,4-tetrahydro-naphthalin, auch «Tonalid» oder «Veritone» genannt (s. DE-PS 2 457 550) und sind ausserdem wertvolle Zwischenprodukte für zahlreiche weitere technische Synthesen. Das gilt auch für das so hergestellte 2,3-Dimethylbuten-2 und das 2,3-Dimethylbutadien.

Beispiel 1

Man benutzt eine Glasapparatur, die aus einem Vierhalskolben mit Rührer, Thermometer und einer Destillationskolonne mit Destillationsaufsatz besteht.

Man setzt ein:

336,6 g Diethylenglykol-n-butyl-tert.-butylether (= DGBB)

112,2 g (1,8 Mol) Kaliumhydroxid t.q. technisch (ca. 90%ig)

492,3 g (4 Mol) 1-Chlor-3,3-dimethylbutan (Neohexylchlorid), 98%ig

Diethylenglykol-n-butyl-tert.-butylether und Kaliumhydroxid werden vorgelegt und unter Rühren und Stickstoffabdeckung auf 220°C erwärmt, wobei sich zwei flüssige Phasen ausbilden. Innerhalb von 6,5 Stunden wird dann Neohexylchlorid zugetropft. Nach Zugabe von ca. 30 ml der Chlorverbindung fällt bei 39 bis 40°C Destillat an. Das Verhältnis Rücklauf zur Abnahme wird zuerst auf 5:1 und nach einer halben Stunde auf 3:1 eingestellt. Bei diesem Rücklaufverhältnis steigt die Kopftemperatur an, und es destilliert dann Wasser mit über, bis nach 7¼ Stunden die Siedetemperatur des Neohexilchlorids (117°C) erreicht ist. Nach 8¼ Stunden ist die 1. Fraktion beendet. Das Sumpfprodukt wird bis auf 160°C abgekühlt, und unter weiterem Rühren fällt bei 133 mbar die Destillationsfraktion 2 an. Die Mengen zeigt die folgende Tabelle:

| Fr. Nr. | Temp. (°C) | | Gewicht g | Druck mbar | Verhältnis Rückl. z. Abnahme |
|---|---|---|---|---|---|
| | Sumpf | Kopf | | | |
| 1 | 220 | 39–117<br>114 | 469 | Normal | 5:1<br>3:1 |
| Kühlfalle z. 1. Fraktion | | | 6 | | |
| 2 | 160–168 | 54–150 | 38 | 133 | 3:1 |
| Kühlfalle z. 2. Fraktion | | | 12 | | |
| Rückstand | | | 429 | | |
| | | | 954 | | |

Die Fraktionen 1 und 2 werden mit den entsprechenden Kühlfallen vereinigt und bei der 1. Fraktion 35 g Wasser abgetrennt. Die in den GC-Analysen erfassten wichtigsten Komponenten dieser beiden Gemische sind in der folgenden Tabelle aufgeführt:

| | Neohexen | Neohexyl-Chlorid | Neohexanol | Dineohexylether | DGBB |
|---|---|---|---|---|---|
| Fraktion 1 und Kühlfalle | 24,1 | 64,4 | 2,2 | – | – |
| Fraktion 2 und Kühlfalle | 0,7 | 15,3 | 3,2 | 20,1 | 36,6 |
| Sumpfphase | – | – | – | – | 91,8 |

Das Sumpfprodukt wird mit 450 g Wasser gerührt und dann die Phasen getrennt: Ölphase 286 g (GC-Analyse s.o.), wässrige Phase 569 m mit 4,66% Kaliumhydroxid und einem Kohlenstoffgehalt von 0,5%.

Aus den o.g. Zahlen errechnet sich der Umsatz an Neohexylchlorid zu ca. 40%. Die Ausbeute an Neohexen, bezogen auf umgesetztes Neohexylchlorid, beträgt ca. 80 Mol-%. Zwei wertvolle Nebenprodukte, Neohexanol und Dineohexylether, entstehen in Ausbeuten, bezogen auf umgesetztes Neohexylchlorid, von 6,9 bzw. 3,1 Mol-%.

Die Neohexen-haltigen Fraktionen werden zusammengegeben und redestilliert. Diese Destillation liefert Neohexen mit 98,5%iger Reinheit nach gaschromatographischer Analyse.

Beispiel 2

Die Durchführung erfolgt wie beim Beispiel 1, anstelle des Kaliumhydroxids wird 1 Mol Kaliumhydroxid und 1 Mol Natriumhydroxid sowie 2 Mol Neohexylchlorid eingesetzt. Hierbei wird ein Umsatz von ca. 50% erreicht. Die Ausbeuten an Neohexen, Neohexanol und Dineohexylether, bezogen auf umgesetztes Neohexalchlorid, liegen bei 70,0, 6,1 bzw. 7,1 Mol-%.

Beispiel 3 (Herstellung des Diglykol-n-butyl-neohexylethers)

Die Durchführung erfolgt wie im Beispiel 1 beschrieben, anstelle des DGBB wird n-Butyldiglykol eingesetzt:

259,5 g (= 2,13 Mol) Neohexylchlorid (99,9%ig).
112,2 g (= 1,8 Mol) Kaliumhydroxid techn. (ca. 90%ig)
336,6 g n-Butyldiglykol (98,6%ig)

Die Reaktionstemperatur wird auf 170°C eingestellt. Auch hierbei bilden sich zwei flüssige, leicht rührbare Phasen.

Der Kohlenstoffgehalt des Abwassers liegt bei 1,3% und wird durch zweimalige Wäsche mit Neohexylchlorid auf 0,7% verringert.

Die nach der Reaktion und der Wasserwäsche erhaltene Ölphase zeigt folgende Zusammensetzung:

n-Butyldiglykol: 47,1%
Diglykol-n-butyl-neohexylether: 50,4%
Hochsieder: 2,5%

Der Umsatz an Neohexylchlorid beträgt 77,7%. Die Ausbeuten, bezogen auf umgesetztes Neohexylchlorid, sind:

Neohexen: 52,7 Mol-%
Neohexanol: 6,5 Mol-%
Dineohexylether: 0,1 Mol-%
Diglykol-n-butyl-neohexylether: 34,2 Mol-%
(= DGBN)

Durch Destillation wird DGBN mit einem Siedepunkt von 145 bis 150°C bei 13 mbar vom n-Butyldiglykol getrennt und mit einer Reinheit von 99,6% gewonnen.

Das H-NMR-Spektrum dieses neuen Ethers zeigt drei verschiedene Signalgruppen entsprechend den Protonenarten, die in der Struktur mit a, b und c gekennzeichnet sind:

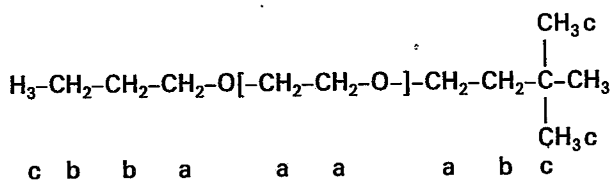

a – Protonen-Signal bei 3,5 ppm
b – Protonen-Signal bei 1,5 ppm
c – Protonen-Signal bei 0,9 ppm

Gemessen in Tetrachlorkohlenstoff gegen Tetramethylsilan als Standard.

Dichte: $d^{20}/4 = 0{,}8751$
Brechungsindex: $n_D^{20} = 1{,}4268$
IR-Banden: 740 $cm^{-1}$ (n-Butyl-Gruppe)
1120 bis 1125 $cm^{-1}$ (R–O–R-Gruppe)

Beispiel 4 (Herstellung des Triglykol-n-butyl-neohexylethers)

Die Durchführung erfolgt wie in Beispiel 3 beschrieben, nur wird anstelle des n-Butyldiglykols n-Butyltriglykol eingesetzt. Es werden folgende Ergebnisse erzielt: Der Kohlenstoffgehalt des Abwassers liegt bei 1,7% und wird durch einmalige Wäsche mit Neohexylchlorid auf 1,3% gesenkt.

Der Umsatz an Neohexylchlorid beträgt 73%. Die Ausbeuten an Wertprodukten, bezogen auf umgesetzten Neohexylchlorid, sind:

Neohexen: 61,0 Mol-%
Neohexanol: 2,4 Mol-%
Triglykol-n-butylneohexylether: 33,2 Mol-%

Auch dieser neue Ether, dessen Siedepunkt bei 13 mbar bei ca. 174°C liegt, wird auf destillativem Wege gereinigt.

Die Lage der Protonensignale im H-NMR-Spektrum stimmt mit dem Spektrum des DGBN gut überein.

Dichte: $d^{20}/4 = 0{,}9102$
Brechungsindex: $n_D^{20} = 1{,}4331$
IR-Banden: 740 $cm^{-1}$ (n-Butylgruppe)
1120 bis 1125 $cm^{-1}$ (R–O–R-Gruppe)

Beispiel 5 (Herstellung des Glykol-n-butyl-neohexylethers)

Die Durchführung erfolgt wie im Beispiel 3 beschrieben, nur wird anstelle des n-Butyldiglykols n-Butyl-glykol eingesetzt. Wegen des relativ niedrigen Siedepunkts wird der unterste Teil der Destillationskolonne mit Luft gekühlt. Es werden ähnliche Ergebnisse erhalten wie bei den Beispielen 3 und 4. Der entstandene neue Glykol-n-butyl-neohexylether hat bei 13 mbar einen Siedepunkt von ca. 92°C und wird durch Destillation in über 99%iger Reinheit erhalten.

Die Lage der Protonensignale im H-NMR-Spektrum dieses Ethers stimmt mit dem Spektrum des DGBN gut überein.

Dichte: $d^{20}/4 = 0{,}8331$
Brechungsindex: $n_D^{20} = 1{,}4187$
IR-Banden: 740 $cm^{-1}$ (n-Butyl-Gruppe)
1120 bis 1125 $cm^{-1}$ (R–O–R-Gruppe)

Beispiel 6

Man benutzt die im Beispiel 1 beschriebene Apparatur und setzt ein:

243 g (= 2 Mol) Neohexylchlorid (99,2%ig)
112,2 g (= 1,8 Mol) Kaliumhydroxid techn. (ca. 90%ig)
336,6 g Diglykol-n-butyl-neohexylether (= DGBN)

DGBN und Kaliumhydroxid werden vorgelegt und unter Rühren und Stickstoffabdeckung auf 200°C erwärmt. Ab ca. 150°C bilden sich zwei flüssige, leicht rührfähige Phasen aus. Die weitere Durchführung und Aufarbeitung erfolgt wie im Beispiel 1 beschrieben.

Es wird folgendes Ergebnis erhalten:

Der Kohlenstoffgehalt des Abwassers liegt bei 1,2%.

Umsatz an Neohexylchlorid: 65%
Ausbeuten, bezogen auf umgesetztes Neohexylchlorid:

Neohexen: 71,3 Mole-%
Neohexanol: 8,7 Mol-%
Dineohexylether: 6,9 Mol-%

Beispiel 7

Die Durchführung erfolgt wie beim Beispiel 6, nur wird die Temperatur von 200 auf 220°C und die Menge an Neohexylchlorid von 2,0 auf 2,5 Mol erhöht.

Ergebnis:

Der Kohlenstoffgehalt des Abwassers liegt bei 0,54%.

Umsatz an Neohexylchlorid: 60,0%
Ausbeuten, bezogen auf umgesetztes Neohexylchlorid:
Neohexen: 64,2 Mol-%
Neohexanol: 10,0 Mol-%
Dineohexylether: 8,8 Mol-%

Beispiel 8

Die Durchführung erfolgt wie beim Beispiel 7, nur wird die Temperatur von 220°C auf 250°C erhöht.

Man erhält folgendes Ergebnis:

Der Kohlenstoffgehalt des Abwassers liegt bei 1,4%.

Der Destillationsrückstand enthält ca. 0,8% n-Butyl-diglykol, das durch Zersetzung von DGBN entstanden ist.

Umsatz an Neohexylchlorid: 40,0%
Ausbeuten, bezogen auf umgesetztes Neohexylchlorid:
Neohexen: 73,8 Mol-%
Neohexanol: 20,2 Mol-%
Dineohexylether: 3,4 Mol-%

Beispiel 9

Man benutzt eine Glasapparatur, die aus einem Vierhalskolben mit Rührer, Thermometer und einer Destillationskolonne mit Destillationsaufsatz besteht.

Man setzt ein:

336,6 g Diethylenglykol-n-butyl-tert.-butylether (= DGBB)
112,2 g (1,8 Mol) Kaliumhydroxid t.q. technisch (ca. 90%ig)
248,1 g (= 2 Mol) Monochloridgemisch (97,2%ig) mit
38,6% 2-Chlor-2,3-dimethylbutan und
58,6% 1-Chlor-2,3-dimethylbutan

Diethylenglykol-n-butyl-tert.-butylether und Kaliumhydroxid werden vorgelegt und unter Rühren und Stickstoffabdeckung auf 200°C erwärmt, wobei sich zwei flüssige Phasen ausbilden. Innerhalb von 2¾ Stunden wird dann Monochloridgemisch zugetropft. Nach Zugabe von ca. 30 ml der Chlorverbindung fällt bei ca. 50°C Destillat an. Das Verhältnis Rücklauf zur Abnahme wird auf 5:1 eingestellt. Die anfallenden Leichtsieder werden, wie in der folgenden Tabelle aufgeführt, abdestilliert, wobei die 1. Fraktion nach 3,5 Std. beendet ist. Dann wird (s. Tabelle) auf 133 mbar umgestellt und die 2. Fraktion abgenommen. Gesamtdauer: 5 Std.

| Fr. Nr. | Temp. (°C) | | Gewicht g | Druck mbar | Verhältnis Rückl. z. Abnahme |
|---|---|---|---|---|---|
| | Sumpf | Kopf | | | |
| 1 | 200 | 51–91 | 188 | Normal | 5:1 |
| 2 | 134–177 | 49–168 | 48 | 133 | 5:1 |
| | | | | | 1:1 |
| Rückstand | | | 439 | | |
| Kühlfalle | | | 6 | | |
| | | | 681 | | |

Die Fraktionen 1 und 2 und das Kühlfallenprodukt werden zusammengegeben und nach Phasentrennung 32 g Wasser abgetrennt. Die Zusammensetzung des Gemischs zeigt die folgende Tabelle unter Produkt A.

Der Rückstand wird mit 300 g Wasser kräftig gerührt und dann die Phasen getrennt:

Obere organische Phase: 305 g
Untere wässrige Phase: 432 g

Der Kohlenstoffgehalt dieses Abwassers liegt bei 0,4%

Die obere Phase wird gaschromatographisch untersucht, siehe folgende Tabelle/Produkt B:

GC-Analysen:

| Chemische Verbindung | Produktgemisch A | Produkt B |
|---|---|---|
| 2,3-Dimethylbuten-1 | 36,0% | - |
| 2,3-Dimethylbuten-2 | 7,6% | - |
| 2-Chlor-2,3-dimethylbutan | 14,1% | - |
| 1-Chlor-2,3-dimethylbutan | 28,7% | - |
| DGBB | 8,8% | 93,9% |
| Hochsieder | < 0,1% | 5,0% |

Aus den angegebenen Zahlen errechnet sich der Umsatz an Monochloriden zu 63,0%. Auf umgesetzte Monochloride bezogen beträgt die Ausbeute an 2,3-Dimethylbuten-1 71,1% und an 2,3-Dimethylbuten-2 15,1%, d.h., das gewünschte α-Olefin entsteht in überraschend hoher Ausbeute.

Das Produktgemisch A liefert nach Redestillation die Olefine in über 98%iger Reinheit nach gaschromatographischer Analyse.

Beispiel 10

Die Durchführung erfolgt wie beim Beispiel 1, anstelle des Kaliumhydroxids wird ein Gemisch von 1 Mol Kaliumhydroxid und 1 Mol Natriumhydroxid sowie 2 Mol Monochloridgemisch mit gleicher Zusammensetzung wie beim Beispiel 1 eingesetzt. Hierbei wird ein Umsatz an Chlorverbindung von 47% erreicht. Die Ausbeuten an 2,3-Dimethylbuten-1 liegt bei 68,0% und an 2,3-Dimethylbuten-2 bei 12,0%.

Beispiel 11 (Herstellung des Diglykol-n-butyl-2,3-dimethylbutylethers)

Die Durchführung erfolgt wie beim Beispiel 1 beschrieben; anstelle des DGBB wird n-Butyldiglykol und anstelle von Kaliumhydroxid wird Natriumhydroxid technisch eingesetzt:

490 g (= 4 Mol) Monochloridgemisch (mit 32,9% 2-Chlor-2,3-dimethylbutan und 65,6% 1-Chlor-2,3-dimethylbutan)
80 g (= 2 Mol) Natriumhydroxid techn.
240 g n-Butyldiglykol

Die Reaktionstemperatur wird auf 170°C eingestellt. Auch hierbei bilden sich zwei flüssige, leicht rührbare Phasen.

Der Kohlenstoffgehalt des Abwassers liegt bei 0,1%.

Die nach der Reaktion und der Wasserwäsche erhaltene Ölphase zeigt u.a. folgende Zusammensetzung:

n-Butyldiglykol 54,0%
Diglykol-n-butyl-2,3-dimethylbutylether 36,4%
Hochsieder 4,9%

Der Umsatz an Monochloriden beträgt ca. 55%, und die Ausbeuten, bezogen auf umgesetzte Chlorverbindungen, sind:
2,3-Dimethylbuten-1 65,7 Mol-%
2,3-Dimethylbuten-2 20,8 Mol-%
Diglykol-n-butyl-2,3-dimethylbutylether 12,4 Mol-% (= DGBDB)

Durch Destillation wird DGBDB mit einem Siedepunkt von 134°C bei 13 mbar in 98,9%iger Reinheit gewonnen.

Das H-NMR-Spektrum dieses neuen Ethers zeigt drei verschiedene Signalgruppen entsprechend den Protonenarten, die in der Struktur mit a, b und c gekennzeichnet sind:

$$H_3C(CH_2)_2CH_2{-}O({-}CH_2{-}CH_2{-}O{-})_2CH_2{-}CH(CH_3){-}CH(CH_3)_2$$

c b a a a a b c b c

a - Protonensignal bei 3,2 bis 3,8 ppm
b - Protonensignal bei 1,3 bis 1,8 ppm
c - Protonensignal bei 0,8 bis 1,9 ppm

Gemessen in Deuterochloroform gegen Tetramethylsilan als Standard.

Dichte: $d^{20}/4 = 0{,}8809$
Brechungsindex: $n_D^{20} = 1{,}4299$
IR-Banden: 740 $cm^{-1}$ (n-Butylgruppe)
1100 bis 1170 $cm^{-1}$ (R–O–R-Gruppe)
1350 bis 1400 $cm^{1}$ ($CH_3$-Gruppe)

Beispiel 12 (Herstellung des Triglykol-n-butyl-2,3-dimethylbutylethers)

Die Durchführung erfolgt wie im Beispiel 3 beschrieben, nur wird anstelle des n-Butyldiglykols n-Butyltriglykol eingesetzt. Es werden folgende Ergebnisse erzielt:

Der Kohlenstoffgehalt des Abwassers liegt bei 1,8%.

Der Umsatz an Monochloridgemisch beträgt 56,0%, und die Ausbeuten sind:

2,3-Dimethylbuten-1 64,5 Mol-%
2,3-Dimethylbuten-2 26,4 Mol-%

Triglykol-n-butyl-2,3-dimethylbutylether 6,5 Mol-%

Dieser neue Ether, dessen Siedepunkt bei 13 mbar bei 170 bis 172°C liegt, wird auf destillativem Weg gereinigt.

Die Lage der Protonensignale im H-NMR-Spektrum stimmt mit dem Spektrum des DGBDB (Beispiel 3) weitgehend überein.

Dichte: $d^{20}/4 = 0{,}9301$
Brechungsindex: $n_D^{20} = 1{,}4362$
IR-Banden: 740 $cm^{-1}$ (n-Butylgruppe)
1100 bis 1170 $cm^{-1}$ (R–O–R-Gruppe)
1350 bis 1400 $cm^{-1}$ ($CH_3$-Gruppe)

Beispiel 13 (Herstellung des Glykol-n-butyl-2,3-dimethylbutylethers)

Die Durchführung erfolgt wie im Beispiel 3 beschrieben, nur wird anstelle des n-Butyldiglykols n-Butylglykol eingesetzt. Wegen des relativ niedrigen Siedepunktes wird der unterste Teil der Destillationskolonne mit Luft gekühlt. Es werden ähnliche Ergebnisse erhalten wie bei den Beispielen 3 und 4.

Der Kohlenstoffgehalt des Abwassers liegt bei 0,9%.

Der entstandene neue Glykol-n-butyl-2,3-dimethylbutylether hat bei 13 mbar einen Siedepunkt von 100 bis 103°C und wird durch Destillation in 98%iger Reinheit erhalten.

Die Lage der Protonensignale im H-NMR-Spektrum dieses Ethers stimmt mit dem Spektrum des DGBDB (Beispiel 3) gut überein.

Dichte: $d^{20}/4 = 0{,}8414$
Brechungsindex: $n_D^{20} = 1{,}4218$
IR-Banden: 740 $cm^{-1}$ (n-Butylgruppe)
1100 bis 1160 $cm^{-1}$ (R–O–R-Gruppe)
1350 bis 1400 $cm^{-1}$ ($CH_3$-Gruppe)

Beispiel 14

Man benutzt die im Beispiel 1 beschriebene Apparatur und setzt ein:

245,0 g (= 2 Mol) Monochloridgemisch (mit 32,9% 2-Chlor-2,3-dimethylbutan und 65,6% 1-Chlor-2,3-dimethylbutan)
124,7 g (= 2 Mol) Kaliumhydroxid techn. (ca. 90%ig)
336,6 g Diglykol-n-butyl-2,3-dimethylbutylether (= DGBDB)

DGBDB und Kaliumhydroxid werden vorgelegt und unter Rühren und Stickstoffabdeckung auf 200°C erwärmt. Ab ca. 150°C bilden sich zwei flüssige, leicht rührfähige Phasen aus. Die weitere Durchführung und Aufarbeitung erfolgt wie im Beispiel 1 beschrieben.

Es wird folgendes Ergebnis erhalten:

Der Kohlenstoffgehalt des Abwassers liegt bei 1,1%.

Umsatz an Monochloridgemisch: 66%

Ausbeuten, bezogen auf umgesetztes Monochloridgemisch:

| | |
|---|---|
| 2,3-Dimethylbuten-1 | 65,2 Mol-% |
| 2,3-Dimethylbuten-2 | 22,5 Mol-% |

Beispiel 15

Die Durchführung erfolgt wie beim Beispiel 6, nur wird die Temperatur von 200 auf 220°C erhöht.

Ergebnisse:

Der Kohlenstoffgehalt des Abwassers liegt bei 1,4%.

Umsatz an Monochloridgemisch: 71%

Ausbeuten, bezogen auf umgesetztes Monochloridgemisch:

| | |
|---|---|
| 2,3-Dimethylbuten-1 | 58,1 Mol-% |
| 2,3-Dimethylbuten-2 | 15,2 Mol-% |

Beispiel 16

Die Durchführung erfolgt wie beim Beispiel 7, nur wird die Temperatur von 220°C auf 250°C erhöht.

Man erhält folgendes Ergebnis:

Der Kohlenstoffgehalt des Abwassers liegt bei 1,8%.

Der Destillationsrückstand enthält ca. 1,2% n-Butyl-diglykol, das durch Zersetzung von DGBDB entstanden ist.

Umsatz an Monochloridgemisch: 65%

Ausbeuten, bezogen auf umgesetztes Monochloridgemisch:

| | |
|---|---|
| 2,3-Dimethylbuten-1 | 52,3 Mol-% |
| 2,3-Dimethylbuten-2 | 11,8 Mol-% |

Beispiel 17

Man benutzt die im Beispiel 1 beschriebene Apparatur und setzt ein rohes schwarzes Produktgemisch ein, das bei der Chlorierung des 2,3-Dimethylbutans nach dem Abdestillieren der Monochlorverbindungen im Sumpf der Kolonne zurückbleibt und vor allem aus Dichloriden des 2,3-Dimethylbutans besteht. Demgemäss liegt der Chlorgehalt bei 42,1% gegenüber dem theoretischen Wert von 45,7%. Wegen der Vielzahl der Komponenten ist eine gaschromatographische Analyse dieses Rohproduktes nicht voll auswertbar; die Analyse zeigt einen Gehalt an Monochloriden von weniger als 1%.

Eingesetzt werden zur Dehydrochlorierung:

273,0 g rohes Dichlor-2,3-dimethylbutan (Chlorgehalt 42,1%)
274,3 g (= 4,4 Mol) Kaliumhydroxid techn. (90%ig)
1097 g DGBDB

Es wird eine Reaktionstemperatur von 200°C eingestellt. Die weitere Durchführung erfolgt wie im Beispiel 1 beschrieben.

Der Kohlenstoffgehalt des Abwassers liegt bei 1,1%.

Die Ausbeute an 2,3-Dimethylbutadien liegt bei 41,9%, bezogen auf eingesetztes Rohprodukt.

Durch Destillation wird das 2,3-Dimethylbutadien in über 97%iger Reinheit erhalten.

## Patentansprüche

1. Verfahren zur Herstellung von 3,3- bzw. 2,3-Dimethylbuten und 2,3-Dimethylbutadien bzw. zur gleichzeitigen Herstellung von 3,3- bzw. 2,3-Dimethylbuten, 2,3-Dimethylbutadien und Glykol- bzw. Polyglykol-n-alkyl-3,3- bzw. -2,3-dimethylbutylether durch Umsetzung von 1-Chlor-3,3-dimethylbutan bzw. 1-Chlor-2,3-dimethylbutan allein oder im Gemisch mit 2-Chlor-2,3-dimethylbutan bzw. von Dichlor-2,3-dimethylbutan mit Alkalihydroxiden, dadurch gekennzeichnet, dass man in einem zweiphasigen flüssigen System Alkalihydroxide in Gegenwart eines Glykol- bzw. Polyglykolethers der Formel 1 in der $R_1$ und $R_2$ die unten angegebene Bedeutung hat, vorlegt und bei Temperaturen von 100 bis 250°C mit 1-Chlor-3,3-dimethylbutan oder 1-Chlor-2,3-dimethylbutan, allein oder im Gemisch mit 2-Chlor-2,3-dimethylbutan, bzw. mit Dichlor-2,3-dimethylbutan umsetzt, wobei man zur Herstellung von 3,3-Dimethylbuten-1 1-Chlor-3,3-dimethylbutan einsetzt, zur Herstellung von 2,3-Dimethylbuten-1 und 2,3-Dimethylbuten-2 1-Chlor-2,3-dimethylbutan allein oder im Gemisch mit 2-Chlor-2,3-dimethylbutan einsetzt und man zur Herstellung von 2,3-Dimethylbutadien Dichlor-2,3-dimethylbutan einsetzt.

$R_1(OCH_2CH_2)_nOR_2$ (1)

$R_1$ = n-Butyl $n = 1, 2, 3$
n-Hexyl
n-Octyl
2-Ethylhexyl
$R_2$ = tert.-Butyl
3,3-Dimethylbutyl oder
2,3-Dimethylbutyl

2. Verfahren nach Anspruch 1, dadurch gekennzeichnet, dass man als Alkalihydroxide Natrium- und/oder Kaliumhydroxid einsetzt.

3. Glykol- und Polyglykol-n-alkyl-3,3- bzw. -2,3-dimethylbutylether der Formel 1.

## Claims

1. A process for the preparation of 3,3- and/or 2,3-dimethylbutene and optionally 2,3-dimethylbutadiene or for the simultaneous preparation of 3,3- and/or 2,3-dimethylbutene and optionally 2,3-dimethylbutadiene and a glycol n-alkyl 3,3- and/or 2,3-dimethylbutyl ether and/or a polyglycol n-alkyl 3,3- and/or 2,3-dimethylbutyl ether by reacting 1-chloro-3,3-dimethylbutane and/or 1-chloro-2,3-dimethylbutane alone or in admixture with 2-chloro-2,3-dimethylbutane and/or dichloro-2,3-dimethylbutane with an alkali metal hydroxide, characterised in that, in a two-phase liquid system, an alkali metal hydroxide is introduced initially in the presence of a glycol ether and/or polyglycol ether of the formula I, where $R_1$ and $R_2$ have the meaning given below, and the mixture is reacted at a temperature from 100 to 250°C with 1-chloro-3,3-dimethylbutane or 1-chloro-2,3-dimethylbutane, alone or in admixture with 2-chloro-2,3-dimethylbutane and/or with dichloro-2,3-dimethylbutane, 1-chloro-3,3-dimethylbutane being employed for the preparation of 3,3-dimethylbutene-1, 1-chloro--2,3-dimethylbutane alone or in admixture with 2-chloro-2,3-dimethylbutane being employed for the preparation of 2,3-dimethylbutene-1 and 2,3-dimethylbutene-2, and dichloro-2,3-dimethylbutane being employed for the preparation of 2,3-dimethylbutadiene.

$R_1(OCH_2CH_2)_nOR_2$ (1)

$R_1$ = n-butyl $n = 1, 2, 3$
n-hexyl
n-octyl
2-ethylhexyl or
$R_2$ = tert.-butyl
3,3-dimethylbutyl or
2,3-dimethylbutyl

2. A process according to claim 1, characterised in that sodium hydroxide and/or potassium hydroxide is used as the alkali metal hydroxide.

3. Glycol n-alkyl 3,3- and/or 2,3-dimethylbutyl ethers and polyglycol n-alkyl 3,3- and/or 2,3-dimethylbutyl ethers of the formula 1.

## Revendications

1. Procédé pour la préparation de 3,3- ou 2,3-diméthyl-butène et de 2,3-diméthyl-butadiène et pour la préparation simultanée de 3,3- ou 2,3-diméthyl-butène, de 2,3-dimèthyl-butadiène et d'éther n-alkyl-3,3- ou -2,3-diméthyl-butylique de glycol ou de polyglycol, par réaction de 1-chloro-3,3-diméthyl-butane ou de 1-chloro-2,3-diméthyl-butane seul ou en mélange avec du 2-chloro-2,3-diméthyl-butane, ou de dichloro-2,3-diméthyl-butane sur des hydroxydes alcalins, caractérisé par le fait que dans un système liquide à deux phases, on place préalablement, en présence d'un éther glycolique ou polyglycolique de la formule 1,

$R_1(OCH_2CH_2)_nOR_2$ (1),

dans laquelle $R_1$ représente un radical n-butyle, n-hexyle, n-octyle, 2-éthylhexyle et $R_2$ représente un radical tertiobutyle, 3,3-diméthyl-butyle ou 2,3-diméthyl-butyle, et n est égal à l'unité, à 2 ou à 3, et qu'on fait réagir le tout, à des températures de 100 à 250°C, sur du 1-chloro-3,3-diméthylbutane ou du 1-chloro-2,3-diméthyl-butane, seul ou en mélange avec du 2-chloro-2,3-diméthylbutane, ou bien sur du dichloro-2,3-diméthylbutane, tandis qu'on utilise, pour préparer du 3,3-diméthyl-butène-(1), du 1-chloro-3,3-diméthyl-butane, qu'on utilise, pour préparer du 2,3-diméthyl-butène-(1) et du 2,3-diméthyl-butène-(2), du 1-chloro-2,3-diméthyl-butane seul ou

en mélange avec du 2-chloro-2,3-diméthyl-butane et que, pour préparer du 2,3-diméthyl-butadiène, on utilise du dichloro-2,3-diméthyl-butane.

2. Procédé selon la revendication 1, caractérisé par le fait qu'on utilise, comme hydroxydes alcalins, l'hydroxyde de sodium et/ou l'hydroxyde de potassium.

3. Les éthers n-alkyl-3,3- ou -2,3-diméthylbutyliques de glycol et de polyglycol de la formule 1.